(19) Europäisches Patentamt

European Patent Office

Office européen des brevets

(11) Veröffentlichungsnummer : **0 391 936 B1**

(12) **EUROPÄISCHE PATENTSCHRIFT**

(45) Veröffentlichungstag der Patentschrift :
27.05.92 Patentblatt 92/22

(21) Anmeldenummer : 88909731.7

(22) Anmeldetag : 17.11.88

(86) Internationale Anmeldenummer :
PCT/DE88/00716

(87) Internationale Veröffentlichungsnummer :
WO 89/04865 01.06.89 Gazette 89/12

(51) Int. Cl.⁵ : **C12M 3/00**

(54) **VORRICHTUNG ZUM KULTIVIEREN VON RUHENDEN ZELLEN IN EINEM BIOREAKTOR.**

(30) Priorität : **16.11.87 DE 3738879**

(43) Veröffentlichungstag der Anmeldung :
**17.10.90 Patentblatt 90/42**

(45) Bekanntmachung des Hinweises auf die
Patenterteilung :
**27.05.92 Patentblatt 92/22**

(84) Benannte Vertragsstaaten :
**AT BE CH DE FR GB IT LI LU NL SE**

(56) Entgegenhaltungen :
**EP-A- 155 237**
**EP-A- 259 109**
**US-A- 4 201 845**

(73) Patentinhaber : **Chmiel, Horst Prof. Dr.-Ing.**
**Paracelsusstrasse 14**
**W-7250 Leonberg-Ramtel (DE)**

(72) Erfinder : **Chmiel, Horst Prof. Dr.-Ing.**
**Paracelsusstrasse 14**
**W-7250 Leonberg-Ramtel (DE)**

(74) Vertreter : **Münich, Wilhelm, Dr. et al**
**Kanzlei Münich, Steinmann, Schiller**
**Willibaldstrasse 36/38**
**W-8000 München 21 (DE)**

EP 0 391 936 B1

## Beschreibung

## Technisches Gebiet

Die Erfindung bezieht sich auf eine Vorrichtung zum kultivieren von ruhenden Zellen in einem Bioreaktor.

## Stand der Technik

Zur Produktion von Wirkstoffen werden heute zunehmend humane, tierische, pflanzliche sowie hybride Zellen im technischen Maßstab kultiviert. Dabei werden zwei grundsätzlich verschiedene Konzepte verfolgt:

Beim "homogenenen Systemen" findet der klassische Rührreaktor Anwendung. Die Zellen befinden sich in Suspension; Nährmedium und Sauerstoff werden durch den Rührvorgang allen Zellen gleichermaßen zur Verfügung gestellt. Die Stoffwechselprodukte und insbesondere das eigentliche Produkt werden durch geeignete Vorrichtungen möglichst kontinuierlich entfernt. Hierzu wird beispielsweise auf die EP-A 0 172 478 verwiesen.

"Heterogene System" findet ihre Anwendung bei den sogenannten "anchorage dependent cells", also jenen Zellen, die nur im Verband oder auf Oberflächen fixiert wachsen.

Die eingangs genannten Zellen zeigen eine hohe Empfindlichkeit gegen
– mechanische Beanspruchung, insbesondere Scherung
– Änderung der Umgebungsbedingungen (pH, Temperatur, $O_2$-Partialdruck)

Außerdem muß die Versorgung mit Nährstoffen und die Entsorgung der Stoffwechselprodukte an jeder Stelle des Bioreaktors gewährleistet sein. Zu diesem Zweck strömt sogenanntes Medium durch den Reaktor, das am Eingang die Nährstoffe, am Ausgang die Stoffwechselprodukte enthält.

Schließlich wird das Verfahren umso wirtschaftlicher, je höher die im Bioreaktor erreichten Zellkonzentrationen sind. Diese Forderungen versucht man in der Praxis auf verschiedenartige Weise zu erreichen.

Ein Weg ist die Fixierung der Zellen auf partikelförmigein der Regel poröse - Träger, die dann vom Medium im Wirbelbett angeströmt werden. Neben der hohen Scherung, die die Zellen dabei erleiden, ergibt sich als weiterer Nachteil, daß die Umgebungsbedingungen und die Nährstoffversorgung der Zellen im unteren Teil des Wirbelbettes anders ist als im oberen. Das analoge Problem findet man heute bei den diversen Membranreaktoren.

Rohr- und Hohlfaser-Membran-Reaktoren benötigen aus wirtschaftlichen Gründen eine Mindestlänge und haben damit konzentrations-, pH- und Temperaturgradienten entlang der Membran, gleichgültig ob die Zellen dabei im Inneren, im Äußeren oder im Ringspalt einer Rohr-bzw. Hohlfaser-Membran wachsen (EP-A 0 123 326, 0 220 650).

Dies trifft gleichermaßen für die in den Europäischen Patentveröffentlichungen 0 155 237, 0 112 155 und 0 180 165 beschriebenen Plattenmodule zu. Wiederum ergeben sich die oben beschriebenen Gradienten mit dem Nachteil, daß an der Seite des Mediumeintritts die Zellen besser versorgt sind und damit besser wachsen und produzieren als auf der Austrittsseite.

## Darstellung der Erfindung

Der Erfindung liegt die Aufgabe zugrunde, eine Vorrichtung zum kultivieren von ruhenden Zellen in einem Bioreaktor derart weiterzubilden, daß insbesondere die Zellen an jeder Stelle des Reaktors gleichermaßen versorgt wie entsorgt werden und an jeder Stelle die gleiche Temperatur, der gleiche pH und die gleichen Gaspartialdrücke herrschen.

Eine erfindungsgemäße Lösung dieser Aufgabe ist mit ihren Weiterbildungen in den Patentansprüchen angegeben.

Erfindungsgemäß wachsen die Zellen auf mit einem biokompatiblen Material beschichteten partikelförmigen Trägern mit Durchmessern zwischen 0,1 mm und 1 mm auf, die in einem 1 mm bis 10 mm dicken Gitter fixiert sind, das von beiden Seiten mit einer porösen Membran verschlossen ist und daß diese Membranen Porendurchmesser haben, die alle Nährstoffe und von den Zellen freigegebenen Produkte hindurchlassen, Zellen und infizierendes Material jedoch zurückhalten.

Weiterbildungen der Erfindung sind in den Unteransprüchen angegeben:

Gemäß Anspruch 2 wird das flächige Gebilde zu einem Wikkelmodul geformt. Da damit das flächige System in mehreren Schichten aufgewickelt ist, bleiben die Gesamtabmessungen des Systems praktikabel sind.

Im Anspruch 3 ist gekennzeichnet, daß das mit Nährstoffen und Sauerstoff angereicherte Medium senkrecht zu einer Membran die fixierten Zellen auf kürzestem Wege durchströmt und die mit den Produkten und

CO$_2$ beladene Flüssigkeit auf kürzestem Weg senkrecht zu einer zweiten Membran den Bioreaktor verläßt.

Im Anspruch 4 ist angegeben, daß das mit Nährstoffen und Sauerstoff angereicherte Medium über einen mit vielen Bohrungen versehenen Wickelmodul gut verteilt über eine Membran auf kürzestem Weg die fixierten Zellen durchströmt, und die mit den Produkten und CO$_2$ beladene Flüssigkeit über eine zweite Membran den Wickelmodul wieder verläßt.

Durch die im Anspruch 5 angegebenen Abstandhalter, die wenigstens ein zwischen den Schichten vorgesehenes weiteres Stützgewebe bildet, wird eine über die ganze Fläche gleichmäßige Flüssigkeitszu- und abfuhr erreicht.

Dabei ist es besonders bevorzugt, wenn - wie im Anspruch 6 gekennzeichnet - zwei Stützgewebe als Abstandshalter vorgesehen sind.

Die im Anspruch 7 angegebene Weiterbildung, gemäß der das Stützgerüst einen Eintritts- und einen Austrittsstutzen zur Befüllung bzw. Entleerung mit partikelförmigen Trägern hat, wird ein kompaktes Modul geschaffen, das, ohne daß es zerlegt werden müßte, regenerierbar ist.

## Kurze Beschreibung der Zeichnung

Die Erfindung wird nachstehend anhand von Ausführungsbeispielen unter Bezugnahme auf die Zeichnung näher beschrieben, in der zeigen:

Fig. 1 den prinzipiellen Aufbau einer erfindungsgemäßen Vorrichtung, und

Fig. 2 eine erfindungsgemäß aufgewickelte Vorrichtung.

## Beschreibung von Ausführungsbeispielen

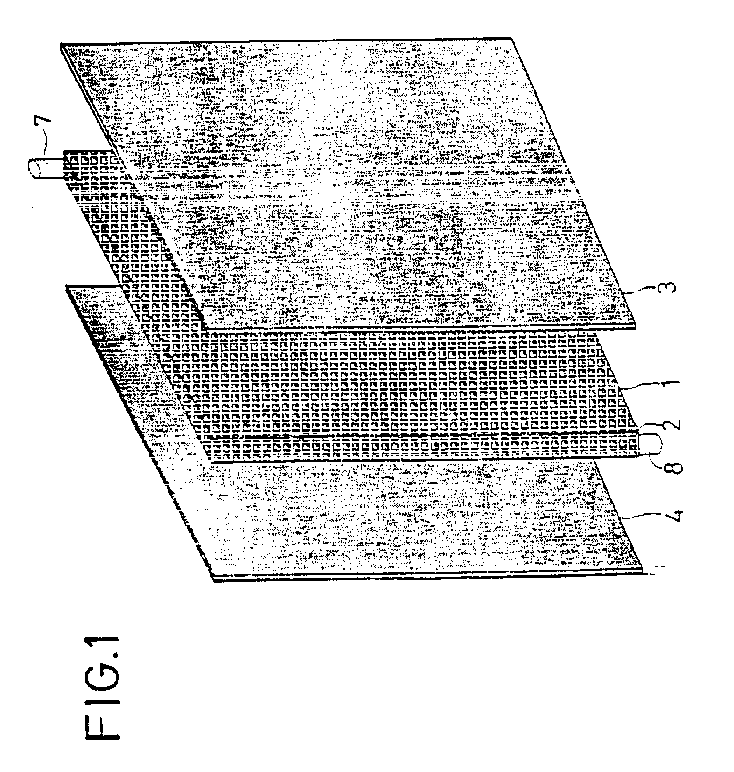

Fig. 1 zeigt, daß die Zellen auf der Oberfläche partikelförmiger Träger (1) aufwachsen, die in einem Gitter (2) fixiert sind. Das nur wenige Millimeter dicke Gitter wird von beiden Seiten mit je einer porösen Membran (3) und (4) verschlossen. Das mit Sauerstoff und Nährstoffen angereicherte Medium strömt senkrecht durch die Membran (3), anschließend auf kürzestem Weg durch das Gitter und damit an den dort auf den partikelförmigen Trägern befindlichen Zellen vorbei. Dabei gibt es Sauerstoff und Nährstoffe an die Zellen ab, nimmt von dort CO$_2$, Stoffwechselprodukte und das eigentliche Produkt auf und verläßt über die Membran (4) den Reaktor.

Die Poren der beiden Membranen (3) und (4) sind so zu wählen, daß sie die zu- bzw. abzuführenden Stoffe nicht zurückhalten. In der Regel werden die Membranen vom Typ "Mikrofiltrationsmembran" (Porendurchmesser 0,1 bis 0,4 μm) sein. Lediglich bei Verwendung proteinfreier (sogenannter serumfreier) Medien kann die Membran (3) eine Ultrafiltrationsmembran (Trenngrenze ca. 10000 bis 100000 Dalton) sein.

Die Oberfläche der Partikel, auf denen die Zellen aufwachsen, müssen biokompatibel sein, d.h. sie müssen möglichst wenig mit den Zellen wechselwirken. Das erreicht man durch geeignete Beschichtung (z.B. mit Albumin). Die Partikeln sollten Durchmesser zwischen 0,1 und 1 mm haben.

Sollen große Mengen an Zellen kultiviert werden, bedeutet das bei dieser Anordnung sehr große Flächen. Die Gesamtabmessung der Vorrichtung kann dennoch praktikabel gehalten werden, indem man das flächige Gebilde aufwickelt.

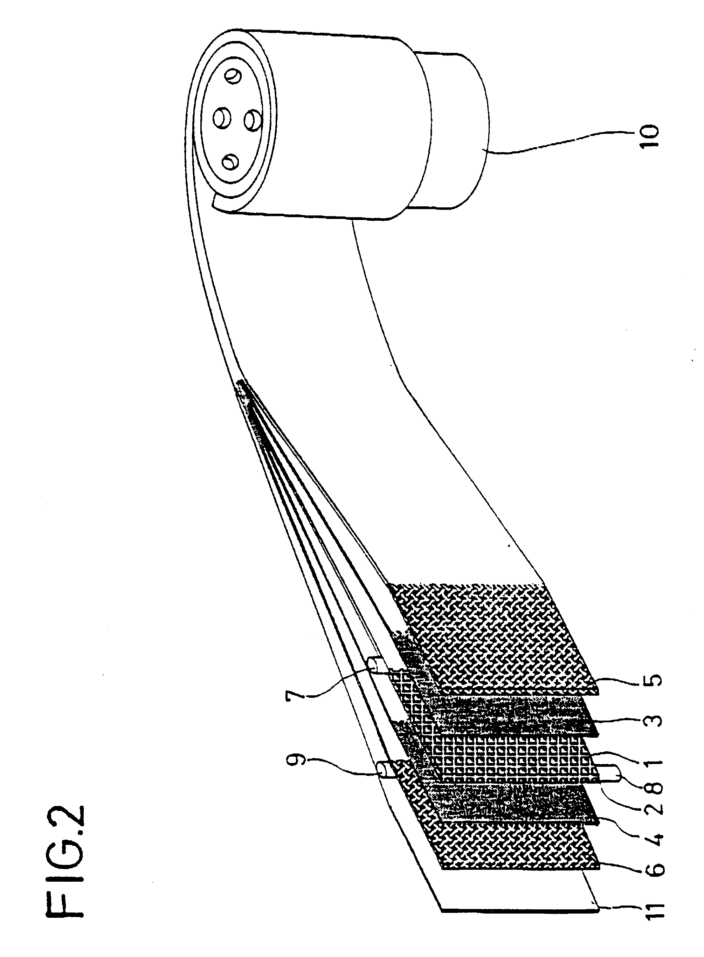

Fig. 2 zeigt einen entsprechenden "Wickelmodul". Dieser Modul weist ein bis zwei weitere Stützgerüste (5) und (6) als Abstandshalter und eine flüssigkeitsdichte Folie (11). Dieser Wickelmodul hat mehrere Anschlüsse: Einen Füllstutzen (8) zum Stützgerüst (2), über den die Befüllung des Moduls mit den partikelförmigen Trägern (1) erfolgt, einen Entleerungsstutzen (7), über den abgestorbene Zellen ausgespült werden können. Das mit Nährstoffen und Sauerstoff angereicherte Medium tritt über den Eintrittsstutzen (10) in den Modul, der aus einem Rohr mit vielen Bohrungen besteht, damit das Medium an allen Stellen gleichmäßig die Membran (3) durchströmt. Die mit Produkt, CO$_2$ und Stoffwechselprodukten beladene Flüssigkeit verläßt - nach Durchtritt durch die Membran (4) - über den Stutzen (9) den Modul.

Damit auf den den Zellen zugewandten Seiten der Membranen keine Proteine adsorbieren, sind diese mit pyrolytischem Kohlenstoff beschichtet. Bei Mikrofiltrationsmembranen empfiehlt sich diese Beschichtung auf beiden Seiten und in den Poren.

## Gewerbliche Anwendbarkeit

Die erfindungsmäße Vorrichtung findet weite Abnwendungen auf allen Gebieten der Biotechnologie.

**Patentansprüche**

1. Vorrichtung zum Kultivieren von ruhenden Zellen in einem Bioreaktor,
dadurch **gekennzeichnet**, daß die Zellen auf mit einem biokompatiblen Material beschichteten partikelförmigen Trägern mit Durchmessern zwischen 0,1 mm und 1 mm aufwachsen, die in einem 1 mm bis 10 mm dicken Gitter fixiert sind, das von beiden Seiten mit einer porösen Membran verschlossen ist, und
daß diese Membranen Porendurchmesser haben, die alle Nährstoffe und von den Zellen freigegebenen Produkte hindurchlassen, Zellen und infizierendes Material jedoch zurückhalten.

2. Vorrichtung nach Anspruch 1,
dadurch **gekennzeichnet**, daß das flächige Gebilde zu einem Wickelmodul geformt wird.

3. Vorrichtung nach Anspruch 1 oder 2,
dadurch **gekennzeichnet**, daß das mit Nährstoffen und Sauerstoff angereicherte Medium senkrecht zu einer Membran die fixierten Zellen auf kürzestem Wege durchströmt und die mit den Produkten und $CO_2$ beladene Flüssigkeit auf kürzestem Weg senkrecht zu einer zweiten Membran den Bioreaktor verläßt.

4. Vorrichtung nach einem der Ansprüche 1 bis 3,
dadurch **gekennzeichnet**, daß das mit Nährstoffen und Sauerstoff angereicherte Medium über einen mit vielen Bohrungen versehenen Wickelmodul gut verteilt über eine Membran auf kürzestem Weg die fixierten Zellen durchströmt, und die mit den Produkten und $CO_2$ beladene Flüssigkeit über eine zweite Membran den Wickelmodul wieder verläßt.

5. Vorrichtung nach einem der Ansprüche 2 bis 4,
dadurch **gekennzeichnet**, daß zwischen den Schichten des Wickelmoduls wenigstens ein weiteres Stützgewebe als Abstandshalter vorgesehen ist, das für eine über die ganze Fläche gleichmäßige Flüssigkeitszu- und abfuhr sorgt.

6. Vorrichtung nach Anspruch 5,
dadurch **gekennzeichnet**, daß zwei Stützgewebe als Abstandshalter vorgesehen sind.

7. Vorrichtung nach einem der Anspüche 2 bis 6,
dadurch **gekennzeichnet**, daß das Stützgerüst einen Eintritts- und einen Austrittsstutzen zur Befüllung bzw. Entleerung mit partikelförmigen Trägern hat.

**Claims**

1. Device for culturing immobilized cells in a bioreactor,
**characterized** in that the cells grow on a particle-shaped substrates coated with a biologically compatible material, having diameters in the range from 0.1 mm to 1 mm, which substrates are immobilized in a lattice being 1mm to 10 mm thick and closed on both sides by a porous diaphragm, and
that these diaphragms have a pore diameter sufficient to let all nutritive substances and products released by the cells pass therethrough whilst cells and infecting material are retained, however.

2. Device according to Claim 1,
**characterized** in that the plane structure is shaped into a coil module.

3. Device according to Claim 1 or 2,
**characterized** in that the medium enriched with nutritive substances and oxygen flows through the immobilized cells along the shortest path and in a direction normal to a diaphram, and that the liquid loaded with the products and $CO_2$ leaves the bioreactor along the shortest path in a direction normal to a second diaphragm.

4. Device according to any of Claims 1 to 3,
**characterized** in that the medium enriched with nutritive substances and oxygen passes through the immobilized cells through a diaphragm along the shortest path, in a pattern well distributed over a coil module provided with a plurality of bores, and that the liquid loaded with the products and $CO_2$ leaves the coil module through a second diaphragm.

5. Device according to any of Claims 2 to 4,
**characterized** in that at least one additional supporting tissue is provided as spacer between the layers of the coil module, which ensures a liquid supply and discharge homogeneous over the entire surface.

6. Device according to Claim 5,
**characterized** in that two supporting tissues are provided as spacers.

7. Device according to any of Claims 2 to 6,
**characterized** in that the supporting structure is provided with an inlet and an outlet tube for the charging or discharge of particle-shaped substrates.

**Revendications**

1. Dispositif de culture de cellules immobilisées dans un bioréacteur,
**caractérisé** en ce que les cellules croissent sur des substrats sous forme de particules, enrobés d'un matériel biologiquement compatible et présentant des diamètres dans la gamme de 0.1 mm à 1 mm, lesquels substrats sont immobilisés dans un réseau à une épaisseur de 1 mm à 10 mm, qui est fermé sur ses deux côtés par une membrane poreuse,
et en ce que ces membranes sont d'un diamètre de pore suffisant pour laisser toutes les matières de culture et tous les produits dégagés par les cellules y passer, pendant que les cellules et du matériel infectieux sont retenus cependant.

2. Dispositif selon la Revendication 1,
**caractérisé** en ce que la structure plane est formée en un module enroulé.

3. Dispositif selon la Revendication 1 ou 2,
**caractérisé** en ce que le milieu enrichi en matières de culture et de l'oxygène s'écoule par les cellules immobilisées par la route la plus courte et en direction perpendiculaire à une membrane, et en ce que le liquide chargé en produits et en $CO_2$ sort du bioréacteur par la route la plus courte en direction perpendiculaire à une deuxième membrane.

4. Dispositif selon une quelconque des Revendications 1 à 3,
**caractérisé** en ce que le milieu enrichi en matières de culture et de l'oxygène s'écoule par les cellules immobilisées par la route la plus courte par une membrane, en une structure bien répartie sur un module enroulé qui est percé d'une pluralité de forages, et en ce que le liquide chargé en produits et en $CO_2$ sort du module enroulé par une deuxième membrane.

5. Dispositif selon une quelconque des Revendications 2 à 4,
**caractérisé** en ce qu'au moins un tissu de support additionnel est disposé en tant qu'un écarteur entre les couches du module enroulé, pour établir une alimentation et décharge du liquide homogène sur toute la surface.

6. Dispositif selon la Revendication 5,
**caractérisé** en ce que deux tissus de support sont disposés en tant que des écarteurs.

7. Dispositif selon une quelconque des Revendications 2 à 6,
**caractérisé** en ce que la structure de support est pourvue d'une tubulure d'entrée et d'une tubulure de sortie pour le chargement ou l'évacuation des substrats sous forme de particules.

FIG.1

EP 0 391 936 B1

# FIG.2

EP 0 391 936 B1